# EUROPEAN PATENT APPLICATION

(11) **EP 4 707 805 A2**
(43) Date of publication of application: **11.03.2026**
(21) Application number: 26151895.5
(22) Date of filing: 16.05.2022
(51) Int. Cl.: G01N 33/532

(54) **ANTI-TSLP NANOBODY AND USE THEREOF**

(30) Priority: 26.01.2022 CN 202210111727
(62) Divisional of application: 22912783.2
(71) Applicant: Shanghai Novamab Biopharmaceuticals Co., Ltd., Pudong New Area, Shanghai 201321 (CN)
(72) Inventor: WAN, Yakun, Shanghai 201321 (CN); LI, Guanghui, Shanghai 201321 (CN); GAI, Junwei, Shanghai 201321 (CN); SHEN, Xiaoning, Shanghai 201321 (CN); ZHU, Min, Shanghai 201321 (CN)
(74) Representative: Mewburn Ellis LLP

(57) **Abstract**

The present invention provides an anti-TSLP nanobody and a use thereof. The present invention provides an anti-TSLP nanobody, and the present invention also provides a coding sequence encoding the above nanobody, corresponding expression vector and host cell capable of expressing the nanobody, and a method for producing the nanobody of the present invention. The nanobody of the present invention has a good TSLP/TSLPR blocking activity; the nanobody of the present invention is expressed using pichia pastoris, and its fermenter expression yield can reach 17-23 g/L.

## Description

### Technical field

The present invention relates to the field of biomedical or biopharmaceutical technology, and more specifically to anti-TSLP nanobodies and their applications.

### Background

In recent years, moderate-to-severe asthma treatment pathways have focused on trying to control the Th2 cell response. Most of the antibody drugs used for the treatment of severe asthma target IgE (omalizumab), IL5 (mepolizumab, Reslizumab), IL5R (benralizumab), and IL4R (Dupilumab) in the Th2 pathway. All of these drugs have good control of hypereosinophilic asthma and are administered subcutaneously or intravenously injection. Approximately one-third of patients with severe asthma do not have features of Th2 inflammatory pathway activation, and there are currently no biologic treatment options for these non-Th2-driven diseases that are not controlled in established standard of care treatments. Therefore, there is an urgent need to develop new treatment pathways.

Thymic stromal lymphopoietin (TSLP) is a short-chain cytokine with a four-helix bundle fold structure and is a member of the IL-2 cytokine family. TSLP is an epithelial cytokine produced in response to pro-inflammatory stimuli, such as allergens in the lungs, viruses and other pathogens and plays a key role in the development and persistence of airway inflammation. TSLP drives the release of downstream Th2 cytokines, including IL-4, IL-5 and IL-13, leading to inflammation and asthma symptoms. TSLP also activates multiple cell types involved in non-Th2 driven inflammation. Thus, early upstream activity of TSLP in the inflammatory cascade response has been identified as a potential target in a broad asthma patient population. Blocking TSLP may prevent the release of pro-inflammatory cytokines from immune cells, thereby preventing asthma exacerbations and improving asthma control, as well as related diseases such as chronic obstructive pulmonary disease.

Nanobody (Nb), or heavy-chain nanobody VHH (variable domain of heavy chain of heavy-chain antibody) - The heavy chain antibodies with naturally deletion of light chains (Heavy-chain antibodies (HCAb)) is present in camels. Nanobodies consisting of only one heavy chain variable region, obtained by cloning its variable region, are the smallest units of stable bindable antigen with full function that can be obtained. Nanobodies are characterized by high stability, good water solubility, simple humanization, high targeting and strong penetration, and play a great function beyond imagination in immune experiments, diagnosis and therapy. Nanobodies are gradually becoming an emerging force in the next generation of antibody therapy.

Therefore, there is an urgent need in this field to develop an anti-TSLP nanobody with better blocking activity, better clinical efficacy, and easy production.

### Summary of the invention

The purpose of the present invention is to provide an anti-TSLP nanobody with better blocking activity, better clinical efficacy, and easy production.

In a first aspect of the present invention, it provides an anti-TSLP nanobody, the complementary determining region CDR region of the VHH chain in the nanobody is one or more selected from the group consisting of:
(1) CDR1 as shown in SEQ ID NO:1, CDR2 as shown in SEQ ID NO:2, and CDR3 as shown in SEQ ID NO: 3;
(2) the CDR1 shown in SEQ ID NO:14, the CDR2 as shown in SEQ ID NO:15, and the CDR3 as shown in SEQ ID NO: 16; and
(3) the CDR1 as shown in SEQ ID NO:27, the CDR2 as shown in SEQ ID NO:28, and the CDR3 as shown in SEQ ID NO: 29.

In another preferred example, the CDR1, CDR2, and CDR3 are separated by the framework regions FR1, FR2, FR3, and FR4 of the VHH chain.

In another preferred embodiment, the VHH chain further comprises a framework region FR, the framework region FR is one or more selected from the group consisting of:
(1) FR1 as shown in SEQ ID NO: 4, FR2 as shown in SEQ ID NO: 5, FR3 as shown in SEQ ID NO: 6, and FR4 as shown in SEQ ID NO: 7;
(2) FR1 as shown in SEQ ID NO: 10, FR2 as shown in SEQ ID NO: 5, FR3 as shown in SEQ ID NO: 6, and FR4 as shown in SEQ ID NO: 11;
(3) FR1 as shown in SEQ ID NO: 17, FR2 as shown in SEQ ID NO: 18, FR3 as shown in SEQ ID NO: 19, and FR4 as shown in SEQ ID NO: 20;
(4) FR1 as shown in SEQ ID NO: 23, FR2 as shown in SEQ ID NO: 18, FR3 as shown in SEQ ID NO: 19, and FR4 as shown in SEQ ID NO: 24;
(5) FR1 as shown in SEQ ID NO: 30, FR2 as shown in SEQ ID NO: 31, FR3 as shown in SEQ ID NO: 32, and FR4 as shown in SEQ ID NO: 33; and
(6) FR1 as shown in SEQ ID NO: 36, FR2 as shown in SEQ ID NO: 31, FR3 as shown in SEQ ID NO: 32, and FR4 as shown in SEQ ID NO: 37.

In another preferred example, the CDR region of the nanobody VHH chain comprises an amino acid sequence having at least 80%, preferably at least 90%, more preferably at least 95%, and even more preferably at least 99% sequence similarity to any of SEQ ID NO:1-3, SEQ ID NO:14-16, and SEQ ID NO:27-29.

In another preferred embodiment, the amino acid sequence of the CDR region of the VHH chain of the nanobody comprises one or more amino acid substitutions, preferably conserved amino acid substitutions, compared to any of SEQ ID NO:1-3, SEQ ID NO:14-16, SEQ ID NO:27-29.

In another preferred embodiment, any one of the above amino acid sequences further comprises a derivative sequence optionally subject to addition, deletion, modification and/or substitution of at least one (e.g. 1-3, preferably, 1-2, more preferably, 1) amino acid and capable of retaining specific binding ability to TSLP

In another preferred embodiment, the nanobody is capable of specifically binding TSLP.

In another preferred embodiment, the nanobody is capable of effectively blocking the interaction of TSLP with TSLPR.

In another preferred embodiment, the TSLP is a TSLP from a human or non-human mammalian.

In another preferred embodiment, the TSLP is a TSLP of a human, mouse, rat, or non-human primate (e.g., monkey).

In another preferred embodiment, the nanobody comprises a humanized antibody, camel-derived antibody, chimeric antibody.

In another preferred example, the amino acid sequence of the VHH chain of the nanobody is selected from the group consisting of SEQ ID NO: 8, SEQ ID NO: 12, SEQ ID NO: 21, SEQ ID NO: 25, SEQ ID NO: 34, SEQ ID NO: 38, and a combination thereof.

In another preferred example, the anti-TSLP nanobody comprises a monomer, a bivalent (bivalent antibody), a quadrivalent (tetravalent antibody), and/or a multivalent (polyvalent antibody).

In another preferred embodiment, the anti-TSLP nanobody comprises one or more VHH chains having an amino acid sequence as shown in SEQ ID NO: 8, SEQ ID NO: 12, SEQ ID NO: 21, SEQ ID NO: 25, SEQ ID NO: 34, or SEQ ID NO: 38.

In another preferred example, the anti-TSLP nanobody comprises two VHH chains having the amino acid sequence as shown in SEQ ID NO: 8, SEQ ID NO: 12, SEQ ID NO: 21, SEQ ID NO: 25, SEQ ID NO: 34, SEQ ID NO: 38.

In another preferred embodiment, the VHH chains are linked to each other by a linker peptide.

In another preferred example, the linker peptide is selected from the sequence: (GₐS_{b})ₓ, wherein a, b, x = 0 or 1 or 2 or 3 or 4 or 5 or 6 or 7 or 8 or 9 or 10 (preferably, a = 4 while b = 1 and x = 4).

In another preferred example, the sequence of the linker peptide is:
GGGGSGGGGSGGGGSGGGGS.

In a second aspect of the present invention, it provides an anti-TSLP antibody which is directed against a TSLP epitope and has an anti-TSLP nanobody as described in the first aspect of the present invention.

In another preferred example, the anti-TSLP antibody comprises one or more anti-TSLP nanobodies.

In another preferred embodiment, the anti-TSLP nanobody comprises a monomer, a bivalent (bivalent antibody), a quadrivalent (tetravalent antibody), and/or a multivalent (polyvalent antibody).

In another preferred embodiment, the anti-TSLP antibody comprises one or more VHH chains having an amino acid sequence as shown in SEQ ID NO: 8, SEQ ID NO: 12, SEQ ID NO: 21, SEQ ID NO: 25, SEQ ID NO: 34, or SEQ ID NO: 38.

In another preferred example, the anti-TSLP antibody comprises two VHH chains having the amino acid sequence as shown in SEQ ID NO: 8, SEQ ID NO: 12, SEQ ID NO: 21, SEQ ID NO: 25, SEQ ID NO: 34, SEQ ID NO: 38.

In another preferred embodiment, the antibody is capable of specifically binding TSLP.

In another preferred embodiment, the antibody is capable of specifically targeting a TSLP protein having the correct spatial structure.

In another preferred embodiment, the antibody is capable of effectively blocking the interaction of TSLP with TSLPR.

In another preferred embodiment, the antibody has an affinity (KD value) for TSLP of less than 3.77 nM.

In a further preferred embodiment, the antibody has a good TSLP/TSLPR blocking activity and the blocking activity is significantly better than the control antibody Tezepelumab, wherein the control antibody Tezepelumab is obtained from AstraZeneca or Amgen.

In another preferred embodiment, the antibody is capable of effectively inhibiting the proliferation of BaF3/TSLPR-IL7R cells with an inhibitory activity superior to that of the control antibody Tezepelumab.

In another preferred embodiment, the antibody is a nanobody.

In a third aspect of the present invention, it provides a polynucleotide encoding a protein selected from the group consisting of: an anti-TSLP nanobody as described in the first aspect of the present invention or an anti-TSLP antibody as described in the second aspect of the present invention.

In another preferred embodiment, the polynucleotide is in a combined form.

In another preferred example, the polynucleotide sequence comprises one or more of the sequences as shown in SEQ ID NO: 9, 13, 22, 26, 35 or 39.

In another preferred example, the polynucleotide comprises RNA, DNA or cDNA.

In a fourth aspect of the present invention, it provides an expression vector, comprising a polynucleotide as described in a third aspect of the present invention.

In another preferred embodiment, the expression vector is selected from the group consisting of: DNA, RNA, viral vector, plasmid, transposon, other gene transfer systems, and a combination thereof.

In another preferred example, the expression vector comprises a viral vector, such as a lentivirus, adenovirus, AAV virus, retrovirus.

In a fifth aspect of the present invention, it provides a host cell containing the expression vector as described in a fourth aspect of the present invention, or having the polynucleotide as described in a third aspect of the present invention integrated in its genome.

In another preferred embodiment, the host cell comprises a prokaryotic cell or a eukaryotic cell.

In another preferred embodiment, the host cell is selected from the group consisting of: Escherichia coli, yeast cell, mammalian cell, phage, and a combination thereof.

In another preferred embodiment, the prokaryotic cell is selected from the group consisting of: Escherichia coli, Bacillus subtilis, Lactobacillus, Streptomyces, Proteus mirabilis, and a combination thereof.

In another preferred embodiment, the eukaryotic cell is selected from the group consisting of: pichia pastoris, Saccharomyces cerevisiae, Schizosaccharomyces pombe, Trichoderma, and a combination thereof.

In another preferred embodiment, the host cell is a pichia pastoris.

In a sixth aspect of the present invention, it provides a method for producing an anti-TSLP nanobody, comprising the steps of:
(a) culturing the host cell as described in the fifth aspect of the present invention under a condition suitable for producing a nanobody, thereby obtaining a culture containing the anti-TSLP nanobody;
(b) isolating or recovering the anti-TSLP nanobody from the culture; and
(c) optionally, purifying and/or modifying the anti-TSLP nanobody obtained in step (b).

In a seventh aspect of the present invention, it provides an immunoconjugate, comprising:
(a) the anti-TSLP nanobody as described in a first aspect of the present invention, or the anti-TSLP antibody as described in a second aspect of the present invention; and
(b) a coupling portion selected from the group consisting of: a detectable marker, a drug, a toxin, a cytokine, a radionuclide, an enzyme, a gold nanoparticle/nanorod, a magnetic nanoparticle, a viral capsid protein or VLP, and a combination thereof.

In another preferred embodiment, the radionuclide comprises:
(i) a diagnostic isotope, the diagnostic isotope being selected from the group consisting of: Tc-99m, Ga-68, F-18, I-123, I-125, I-131, In-111, Ga-67, Cu-64, Zr-89, C-11, Lu-177, Re-188, and a combination thereof; and/or
(ii) a therapeutic isotope, the therapeutic isotope being selected from the group consisting of: Lu-177, Y-90, Ac-225, As-211, Bi-212, Bi-213, Cs-137, Cr-51, Co-60, Dy-165, Er-169, Fm-255, Au-198, Ho-166, 1-125, I-131 , Ir-192, Fe-59, Pb-212, Mo-99, Pd-103, P-32, K-42, Re-186, Re-188, Sm-153, Ra223, Ru-106, Na24, Sr89, Tb-149, Th-227, Xe-133, Yb-169, Yb-177, and a combination thereof.

In another preferred example, the coupling portion is a drug or toxin.

In another preferred example, the drug is a cytotoxic drug.

In another preferred example, the cytotoxic drug is selected from the group consisting of: an antitubulin drug, DNA minor groove binding agent, DNA replication inhibitor, alkylating agent, antibiotic, folic acid antagonists, antimetabolites, chemosensitizer, topoisomerase inhibitor, vinca alkaloid, and a combination thereof.

In another preferred embodiment, examples of particularly useful cytotoxic agents include, for example, DNA minor groove binding reagents, DNA alkylation reagents, and tubulin inhibitors, typical cytotoxic agents include, for example, auristatins, camptothecins, duocarmycins, etoposides, maytansines and maytansinoids (e.g., DM1 and DM4), taxanes, benzodiazepines, or benzodiazepine containing drugs (e.g., pyrrolo[1,4] benzodiazepines (PBDs), indolinobenzodiazepines, and oxazolidinobenzodiazepines), vinca alkaloids, and combinations thereof.

In another preferred example, the toxin is selected from the group consisting of: auristatin (e.g., auristatin E, auristatin F, MMAE, and MMAF), aureomycin, maytansinoid, ricin, ricin A-chain, combretastatin, duocarmycin, dolastatin, doxorubicin, daunorubicin, paclitaxel, cisplatin, cc1065, ethidium bromide, mitomycin, etoposide, tenoposide, vincristine, vincaleukoblastinum, colchicine, dihydroxyanthracin diketone, actinomycin, diphtheria toxin, Pseudomonas exotoxin (PE) A, PE40, abrin, abrin A chain, modeccin A chain, α- sarcina, gelonin, mitogellin, retstrictocin, phenomycin, enomycin, curicin, crotin, Calicheamicin, Sapaonaria officinalis inhibitor, glucocorticoid, and a combination thereof.

In another preferred example, the coupling portion is a detectable marker.

In another preferred embodiment, the coupling portion is selected from the group consisting of: a fluorescent or luminescent marker, radioactive marker, MRI (magnetic resonance imaging) or CT (computed tomography) contrast agent, or enzyme capable of producing a detectable product, radionuclide, biotoxin, cytokine (e.g. IL-2, etc.), antibody, antibody Fc fragment, antibody scFv fragment, gold nanoparticle/nanorod, viral particle, lipidosome, magnetic nanoparticle, prodrug-activating enzyme (e.g., DT- diaphorase (DTD) or biphenyl hydrolase-like protein (BPHL)) or nanoparticle in any form.

In an eighth aspect of the present invention, it provides a multispecific antibody, the multispecific antibody comprising: an anti-TSLP nanobody as described in a first aspect of the present invention, or an anti-TSLP antibody as described in a second aspect of the present invention.

In another preferred embodiment, the multispecific antibody further comprises an Fc segment of the antibody.

In a ninth aspect of the present invention, it provides a recombinant protein, the recombinant protein having:
(i) an anti-TSLP nanobody as described in a first aspect of the present invention, or an anti-TSLP antibody as described in a second aspect of the present invention; and
(ii) optionally a tag sequence to assist in the expression and/or purification.

In a further preferred example, the tag sequence comprises an Fc tag, an HA tag and a 6His tag.

In a further preferred example, the recombinant protein specifically binds to a TSLP protein.

In a tenth aspect of the present invention, it provides a pharmaceutical composition, the pharmaceutical composition comprising:
(i) an anti-TSLP nanobody as described in the first aspect of the present invention, or an anti-TSLP antibody as described in the second aspect of the present invention, or an immunoconjugate as described in the seventh aspect of the present invention or a multispecific antibody as described in the eighth aspect of the present invention or a recombinant protein as described in the ninth aspect of the present invention; and
(ii) a pharmaceutically acceptable vector.

In a further preferred example, the coupling portion of the immunoconjugate is a drug, a toxin, and/or a therapeutic isotope.

In a further preferred example, the pharmaceutical composition further contains other drugs for the treatment of an immune system disease or a neoplastic disease.

In a further preferred embodiment, other drugs for the treatment of an immune system disease or a neoplastic disease are selected from the group consisting of budesonide, fluticasone, beclomethasone, mometasone furoate, salbutamol, theophylline, formoterol, tiotropium bromide, salazosulfapyridine, methotrexate, cyclophosphamide, fluorouracil, bleomycin, anastrozole, and a combination thereof.

In a further preferred example, the pharmaceutical composition is used in the preparation of a drug for the prevention and/or treatment of a disease or condition associated with TSLP.

In another preferred embodiment, the disease or condition associated with TSLP comprises an immune system disease or an oncological disease.

In a further preferred example, the immune system disease is selected from the group consisting of: asthma, atopic dermatitis, chronic obstructive pulmonary disease (COPD), allergic conjunctivitis, food allergy, ulcerative colitis, Crohn's disease, rhinitis, ankylosing spondylitis, systemic lupus erythematosus, rheumatoid arthritis, Hypersensitivity Pneumonities, allergic granulomatous angiitis, nasal polyposis, and a combination thereof.

In a further preferred example, the oncological disease is selected from the group consisting of: breast cancer, pancreatic cancer, cervical cancer, multiple myeloma, colorectal cancer, lung cancer, thyroid cancer, ovarian cancer, liver cancer, and a combination thereof.

In an eleventh aspect of the present invention, it provides a use of an anti-TSLP nanobody as described in the first aspect of the present invention, an anti-TSLP antibody as described in the second aspect of the present invention, an immunoconjugate as described in the seventh aspect of the present invention or a multispecific antibody as described in the eighth aspect of the present invention or a recombinant protein as described in the ninth aspect of the present invention or a pharmaceutical composition as described in the tenth aspect of the present invention (a) for the preparation of a drug for the prevention and/or treatment of a disease associated with TSLP; and/or (b) for the preparation of a reagent, a detection plate or a kit for the detection of TSLP.

In a further preferred example, the disease or condition associated with TSLP comprises an immune system disease or an oncological disease.

In a further preferred example, the immune system disease is selected from the group consisting of: asthma, atopic dermatitis, chronic obstructive pulmonary disease (COPD), allergic conjunctivitis, food allergy, ulcerative colitis, Crohn's disease, rhinitis, ankylosing spondylitis, systemic lupus erythematosus, rheumatoid arthritis, Hypersensitivity Pneumonities, allergic granulomatous angiitis, nasal polyposis, and a combination thereof.

In a further preferred example, the oncological disease is selected from the group consisting of: breast cancer, pancreatic cancer, cervical cancer, multiple myeloma, colorectal cancer, lung cancer, thyroid cancer, ovarian cancer, liver cancer, and a combination thereof.

In a further preferred example, the TSLP is a human TSLP.

In a further preferred example, the reagent is a diagnostic reagent.

In a further preferred example, the diagnostic reagent is a contrast agent.

In a further preferred embodiment, the reagent is used to detect TSLP protein or a fragment thereof in a sample.

In a further preferred embodiment, the detection comprises a flow cytometer detection, a cellular immunofluorescence assay.

In a further preferred example, the use is diagnostic and/or non-diagnostic, and/or therapeutic and/or non-therapeutic.

In a twelfth aspect of the present invention, it provides a method for detecting TSLP protein in a sample, comprising the steps of:
(1) contacting the sample with an anti-TSLP nanobody as described in the first aspect of the invention, or an anti-TSLP antibody as described in the second aspect of the invention, or an immunoconjugate as described in the seventh aspect of the invention or a multispecific antibody as described in the eighth aspect of the invention or a recombinant protein as described in the ninth aspect of the invention; and
(2) detecting the formation of an antigen-antibody complex, wherein the formation of a complex indicates the presence of TSLP protein in the sample.

In a further preferred example, the method is non-diagnostic and non-therapeutic.

In a thirteenth aspect of the present invention, it provides a TSLP protein detection reagent, comprising:
(i) an anti-TSLP nanobody as described in the first aspect of the present invention, or an anti-TSLP antibody as described in the second aspect of the present invention, or an immunoconjugate as described in the seventh aspect of the present invention or a multispecific antibody as described in the eighth aspect of the present invention or a recombinant protein as described in the ninth aspect of the present invention; and
(ii) a vector that is acceptable for the detection.

In a further preferred embodiment, the coupling portion of the immunoconjugate is a diagnostic isotope.

In a further preferred embodiment, the vector that is acceptable for the detection is a non-toxic, inert aqueous carrier medium.

In a further preferred embodiment, the detection reagent is one or more reagents selected from the group consisting of: an isotopic tracer, a contrast agent, a flow cytometry detection reagent, a cellular immunofluorescence detection reagent, a magnetic nanoparticle and a developer.

In a further preferred embodiment, the detection reagent is used for in vivo detection.

In a further preferred embodiment, the dosage form of the detection reagent is liquid or powder (e.g., water, injection, lyophilized powder, tablet, apomorphine SL, aerosol).

In a fourteenth aspect of the present invention, it provides a kit for detecting a TSLP protein, containing an immunoconjugate as described in the seventh aspect of the present invention or a detection reagent as described in the thirteenth aspect of the present invention, and instructions.

In a further preferred example, the instructions describe that the kit is for non-invasive detection of TSLP expression in a subject to be tested.

In a fifteenth aspect of the present invention, it provides a use of an immunoconjugate as described in the seventh aspect of the present invention for the preparation of a contrast agent for the in vivo detection of TSLP protein.

In a further preferred embodiment, the detection is used for the diagnosis or prognosis of a disease or condition associated with TSLP.

In a sixteenth aspect of the present invention, it provides a method for treating a disease, comprising, administering to a subject in need thereof an anti-TSLP nanobody as described in the first aspect of the present invention, an anti-TSLP antibody as described in the second aspect of the present invention, an immunoconjugate as described in the seventh aspect of the present invention or a multispecific antibody as described in the eighth aspect of the present invention or a recombinant protein as described in the ninth aspect of the present invention or a pharmaceutical composition as described in the tenth aspect of the present invention.

In a further preferred embodiment, the subject comprises a human or non-human mammal.

In a further preferred example, the non-human mammal comprises a rodent (e.g., mouse or rat, rabbit), a non-human primate (e.g., monkey).

It should be understood that within the scope of the present invention, the above-mentioned technical features of the present invention and the technical features described in detail below (such as embodiments) can be combined with each other to form a new or preferred technical solution. Due to space constraints, I will not repeat them here.

### Description of Drawings

Figures 1A and 1B show the results of flow cytometry blocking activity assays performed on 31 candidate antibodies. The results show that 14 of the 31 candidate antibodies show significantly better blocking activity than the control antibody Tezepelumab.
Figure 2 shows the results of the binding kinetics assay of the 14 blocking TSLP nanobodies.
Figure 3 shows the results of SDS-PAGE of shake flask expression supernatants of the bivalent single domain antibodies Bi-HuNb5-31, Bi-HuNb7-54, and Bi-HuNb10-63 in pichia pastoris with yields of 475ug/mL, 310ug/mL, and 510ug/mL, respectively.
Figure 4 shows the results of ELISA for humanized bivalent antibody blocking activity. The results show that the blocking activity of the three strains of humanized bivalent antibodies is significantly better than that of the control antibody Tezepelumab.
Figure 5 shows the results of the proliferation inhibition of BaF3/TSLPR-IL7R cells by humanized bivalent antibodies. The results show that two of the humanized antibodies inhibited the pSTAT5 signaling pathway in BaF3/TSLPR-IL7R cells better than the control antibody Tezepelumab.

### DETAILED DESCRIPTION

For the first time, a class of anti-TSLP nanobodies is unexpectedly identified and experimental results show that the nanobodies of the present invention can effectively block the interaction between TSLP and TSLPR and the blocking activity is significantly better than that of the control antibody Tezepelumab; the nanobodies of the present invention can effectively inhibit the proliferation of Baf3/TSLPR-IL7R cells and the inhibitory activity is better than that of the control antibody Tezepelumab; the fermentor expression yield of the nanobodies of the present invention in pichia pastoris can reach 17-23 g/L, which is significantly higher than the industry level. On this basis, the present inventors have completed the present invention.

### Terms

As used herein, the terms "nanobody of the present invention", "nanobodies of the present invention", "anti-TSLP nanobody of the present invention", " TSLP nanobodies of the present invention", "anti-TSLP nanobodies", "TSLP nanobodies" have the same meaning and are used interchangeably to refer to nanobodies that specifically recognize and bind to TSLP (including human TSLP).

As used herein, the term "antibody" or "immunoglobulin" is a heterotetramerization glycoprotein of approximately 150,000 daltons having the same structural characteristics and consisting of two identical light chains (L) and two identical heavy chains (H). Each light chain is linked to the heavy chain by a covalent disulphide bond, while different immunoglobulin isoforms have different numbers of disulphide bonds between the heavy chains. Each heavy and light chain also has regularly spaced intrachain disulfide bond. Each heavy chain has a variable region (VH) at one end, followed by multiple constant regions. Each light chain has a variable region (VL) at one end and a constant region at the other; the constant region of the light chain is opposite the first constant region of the heavy chain, and the variable region of the light chain is opposite the variable region of the heavy chain. Special amino acid residues form the interface between the variable region of the light chain and the variable region of the heavy chain.

As used herein, the terms "single domain", "VHH", "nanobody", "heavy chain antibody "(single domain antibody, sdAb, or nanobody) have the same meaning and are used interchangeably to refer to the cloning of the variable region of an antibody heavy chain and the construction of a nanobody (VHH) consisting of only one variable region of the heavy chain, which is the smallest antigen-binding fragment with full functionality. Nanobodies (VHH) consisting of only one heavy chain variable region are usually constructed by cloning the variable region of the heavy chain of an antibody after obtaining an antibody with a natural deletion of the light chain and the heavy chain constant region 1 (CH1).

As used herein, the term "variable" indicates that certain portions of the variable region of an antibody differ in sequence, which forms the binding and specificity of various specific antibodies to their particular antigen. However, variability is not uniformly distributed throughout the variable region of the antibody. It is concentrated in three segments of the light and heavy chain variable regions known as the complementary determining region (CDR) or hypervariable region. The more conserved part of the variable region is called the framework region (FR). The variable regions of the natural heavy and light chains each contain four FR regions, which are in a roughly b-folded conformation, linked by three CDRs that form a linking loop and in some cases can form a partially b-folded structure. The CDRs in each chain are held close together by the FR region and together with the CDR of the other chain to form the antigen-binding site of the antibody (see Kabat et al, NIH Publ. No. 91-3242, vol. I, pp. 647-669 (1991)). Constant regions are not directly involved in antibody-antigen binding, but they exhibit different effector functions, such as involvement in antibody-dependent cytotoxicity.

As known to those skilled in the art, immunoconjugates and fusion expression products include: conjugates formed by the binding of drugs, toxins, cytokines, radionuclides, enzymes and other diagnostic or therapeutic molecules to the antibodies of the invention or fragments thereof. The invention also includes cell surface markers or antigens that bind to the anti-TSLP antibodies or fragments thereof.

As used herein, the terms "heavy chain variable region" and "VH" are used interchangeably.

As used herein, the term "variable region" is used interchangeably with the term "complementarity determining region (CDR)".

In a preferred embodiment of the present invention, the heavy chain variable region of the antibody comprises three complementarity determining regions CDR1, CDR2, and CDR3.

In a preferred embodiment of the present invention, the heavy chain of the antibody comprises the heavy chain variable region and the heavy chain constant region as described above.

In the present invention, the terms "antibody of the invention", "protein of the invention", or "polypeptide of the invention" are used interchangeably and all refer to a polypeptide that specifically binds to a TSLP protein, such as proteins or polypeptides having a heavy chain variable region. They may or may not contain the starting methionine.

The present invention also provides other proteins or fusion expression products having antibodies of the invention. Specifically, the invention includes any proteins or protein conjugates and fusion expression products (i.e., immunoconjugates and fusion expression products) having a heavy chain containing a variable region, provided that the variable region is identical or at least 90% homologous, preferably at least 95% homologous, to the heavy chain variable region of the antibodies of the invention.

In general, the antigen-binding properties of antibodies can be described by three specific regions, called variable regions (CDRs), located in the variable region of the heavy chain, spacing the segment into four framework regions (FRs). The amino acid sequences of the four FRs are relatively conservative and are not directly involved in the binding reaction. These CDRs form a ring-like structure and are spatially structured close to each other by the β-fold formed by the FRs between them. The CDRs on the heavy chain and the CDRs on the corresponding light chain form the antigen-binding site of the antibody. It is possible to determine which amino acids constitutes the FR or CDR region by comparing the amino acid sequences of antibodies of the same type.

The variable regions of the heavy chains of the antibodies of the present invention are of particular interest since at least part of them involves binding antigen. The present invention therefore includes those molecules having a variable region of the heavy chain of an antibody with a CDR, provided that its CDR has more than 90% (preferably more than 95%, most preferably more than 98%) homology with the CDR identified herein.

The invention includes not only intact antibodies but also fragments of immunologically active antibodies or fusion proteins formed by antibodies with other sequences. Thus, the invention also includes fragments, derivatives and analogues of the antibodies.

As used herein, the terms "fragments", "derivatives" and "analogues" refer to polypeptides that retain substantially the same biological function or activity as the antibodies of the present invention. A polypeptide fragment, derivative or analogue of the present invention may be (i) a polypeptide having one or more conserved or non-conserved amino acid residues (preferably conserved amino acid residues) substituted, where such substituted amino acid residues may or may not be encoded by the genetic code, or (ii) a polypeptide having a substituent group in one or more amino acid residues, or (iii) a polypeptide formed by fusing a mature polypeptide with another compound (e.g. a compound that extends the half-life of the polypeptide, such as polyethylene glycol), or (iv) a polypeptide formed by the fusion of additional amino acid sequences to this polypeptide sequence (e.g. a lead or secreted sequence or a sequence used to purify this polypeptide or a proteinogen sequence, or a fusion protein formed with a 6His tag). According to the teachings herein, these fragments, derivatives and analogues are within the scope of what is well known to those skilled in the art.

Antibodies of the present invention refer to polypeptides having TSLP binding activity and comprising the CDR region as described above. The term also includes variant forms of polypeptides having the same function as the antibodies of the present invention and comprising the CDR region as described above. These variant forms include, but are not limited to, deletion, insertion and/or substitution of one or more (typically 1-50, preferably 1-30, more preferably 1-20, most preferably 1-10) amino acids, and addition of one or more (typically up to 20, preferably up to 10, more preferably up to 5) amino acids to the C-terminus and/or N-terminus. For example, in the art, substitution with amino acids of similar or close properties does not normally alter the function of the protein. For example, the addition of one or more amino acids to the C-terminus and/or N-terminus does not normally alter the function of the protein. The term also includes active fragments and active derivatives of antibodies of the present invention.

Variant forms of the polypeptide include: homologous sequences, conserved variants, allelic variants, natural mutants, induced mutants, proteins encoded by DNA that can hybridize with the DNA encoding the antibody of the invention under high or low stringency conditions, and polypeptides or proteins obtained using antiserum against the antibody of the invention.

The invention also provides other polypeptides, such as fusion proteins comprising nanobodies or fragments thereof. In addition to almost full-length polypeptides, the present invention also includes fragments of nanobodies of the present invention. Typically, the fragment has at least about 50 contiguous amino acids of the antibody of the invention, preferably at least about 50 contiguous amino acids, more preferably at least about 80 contiguous amino acids, and most preferably at least about 100 contiguous amino acids.

For the purposes of the present invention, "conserved variant of the antibody of the invention" means a polypeptide formed by the substitution of up to 10, preferably up to 8, more preferably still up to 5, most preferably up to 3 amino acids by amino acids of similar or close nature compared to the amino acid sequence of the antibody of the invention. These conserved variant polypeptides are preferably produced by amino acid substitution according to Table A.

**Table A**

| The initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The invention also provides polynucleotide molecules encoding the above-mentioned antibodies or fragments thereof or fusion proteins thereof. The polynucleotides of the invention may be in the form of DNA or RNA. the form of DNA includes cDNA, genomic DNA or synthetic DNA. DNA may be single-stranded or double-stranded. DNA may be coding or non-coding stranded.

The polynucleotides encoding the mature polypeptide of the invention include: coding sequences encoding only the mature polypeptide; coding sequences and various additional coding sequences of the mature polypeptide; coding sequences (and optionally additional coding sequences) of the mature polypeptide and non-coding sequences.

The term "polynucleotide encoding a polypeptide" may include a polynucleotide encoding such a polypeptide, or may also include additional polynucleotides encoding and/or non-encoding sequence.

The invention also relates to polynucleotides that hybridize with the above-mentioned sequences and have at least 50%, more preferably at least 70%, most preferably at least 80% identity between the two sequences. The present invention relates in particular to polynucleotides which are hybridizable with the polynucleotides described herein under stringent conditions. In the context of the present invention, "stringent conditions" means: (1) hybridization and elution at lower ionic strengths and higher temperatures, e.g., 0.2 x SSC, 0.1% SDS, 60°C; or (2) hybridization with denaturing agents, e.g., 50% (v/v) formamide, 0.1% calf serum/0.1% Ficoll 42°C, etc.; or (3) hybridization occurs only when the identity between the two sequences is at least 90%, preferably 95% or more. Moreover, the hybridizable polynucleotide-encoded polypeptides has the same biological function and activity as the mature polypeptide.

The full-length nucleotide sequences of the antibodies of the present invention or fragments thereof can usually be obtained by PCR amplification, by recombinant methods or by synthetic methods. One possible method is to synthesize the sequence in question by synthetic methods, especially when the fragments are short in length. Often, very long sequences can be obtained by first synthesizing a number of small fragments and then ligating them. Alternatively, the coding sequence of the heavy chain can be fused with an expression tag (e.g., 6His) to form a fusion protein.

Once the sequence in question has been obtained, it can be obtained in large quantities by recombinant methods. This is usually done by cloning into a vector, transferring into cells and then isolating the sequence in question from the proliferated host cells by conventional methods. The biomolecules (nucleic acids, proteins, etc.) covered by the present invention include those in isolated form.

It is now possible to obtain a DNA sequence encoding a protein of the invention (or a fragment thereof, or a derivative thereof) entirely by chemical synthesis. This DNA sequence can then be introduced into various existing DNA molecules (or e.g., vectors) and cells known in the art. In addition, mutations can be introduced into the protein sequence of the invention by chemical synthesis.

The invention also relates to vectors comprising suitable DNA sequences as described above and suitable promoters or control sequences. These vectors can be used to transform a suitable host cell to enable it to express a protein.

The host cell may be a prokaryotic cell, such as a bacterial cell; or a lower eukaryotic cell, such as a yeast cell; or a higher eukaryotic cell, such as a mammalian cell. Representative examples are: E. coli, Streptomyces; bacterial cells of salmonella typhimurium; fungal cells such as yeast; insect cells of Drosophila S2 or Sf9; animal cells of CHO, COS7, 293 cells, etc.

Transformation of host cells with recombinant DNA can be carried out by conventional techniques known to those skilled in the art. When the host is a prokaryote such as E. coli, competent cell capable of taking up DNA can be harvested after an exponential growth period and treated with the CaCl₂ method, used steps are well known in the art. Another method is to use MgCl₂. If desired, transformation can also be carried out by electroporation. When the host is a eukaryote, the following DNA transfection methods are available: calcium phosphate co-precipitation, conventional mechanical methods such as microinjection, electroporation, liposome packaging, etc.

The transformants obtained can be cultured by conventional methods to express the polypeptide encoded by the gene of the invention. Depending on the host cells used, the medium used in culture can be selected from a variety of conventional medium. The culture is carried out under conditions suitable for the growth of the host cells. After the host cells have grown to an appropriate cell density, the cells are cultured for a further period of time using a suitable method (e.g., temperature shift or chemical induction) to induce the selected promoter.

The recombinant polypeptide in the above method can be expressed intracellularly, or on the cell membrane, or secreted extracellularly. If desired, the recombinant protein can be isolated and purified by various isolation methods using its physical, chemical and other properties. These methods are well known to those skilled in the art. Examples of these methods include, but are not limited to: conventional renaturation treatment, treatment with protein precipitating agents (salting methods), centrifugation, osmotic breakage, ultra-processing, ultra-centrifugation, molecular sieve chromatography (gel filtration), adsorption chromatography, ion exchange chromatography, high performance liquid chromatography (HPLC) and various other liquid phase chromatography techniques and combinations of these methods.

The antibodies of the invention can be used alone or in combination or coupling with detectable markers (for diagnostic purposes), therapeutic agents, PK (protein kinase) modified fractions or any combination of these.

Detectable markers for diagnostic purposes include, but are not limited to, fluorescent or luminescent markers, radioactive markers, MRI (magnetic resonance imaging) or CT (computed tomography) contrast agents, or enzymes capable of producing detectable products.

Therapeutic agents that can be bound or coupled to antibodies of the present invention include, but are not limited to: 1. radionuclides; 2. biotoxins; 3. cytokines such as IL-2, etc.; 4. gold nanoparticles/nanorods; 5. viral particles; 6. liposomes; 7. magnetic nanoparticles; 8. prodrug-activating enzymes (e.g., DT- diaphorase (DTD) or biphenyl hydrolase-like protein (BPHL)), etc.

### Thymic stromal lymphopoietin (TSLP)

Thymic stromal lymphopoietin is a short-chain cytokine with a four-helix bundle fold structure and is a member of the IL-2 cytokine family. As a novel cytokine, TSLP is normally expressed in epithelial cells on the surface of the lung, skin and intestinal barriers. Animal studies have shown that TSLP is highly expressed in the lungs of mice in an allergen-induced asthma model, while TSLP receptor-deficient mice exhibit significantly reduced asthma and lung-specific TSLP transgenic mice show airway inflammation and hyperresponsiveness with Th2-type inflammation and increased IgE. Further studies suggest that TSLP activates bone marrow-derived dendritic cells and upregulates co-stimulatory molecules to produce the Th2 cell chemokine CCL17. Thus, TSLP is an important factor and necessary for the initiation of allergic airway inflammation. It is an upstream regulator of multiple inflammatory pathways in various diseases, including asthma, and is essential for the development and persistence of airway inflammation. In addition, studies have shown that TSLP is also a cytokine implicated in the pathogenesis of atopic dermatitis (AD). More importantly, researchers have found that TSLP levels are elevated in licensed different tumor types, and that TSLP in turn is able to induce tumor cells to express another protein called BCL-2, which protects tumors from death. TSLP is therefore also essential for tumor survival.

### Thymic stromal lymphopoietin receptor (TSLPR)

TSLPR receptors are type I transmembrane proteins that are members of the haematopoietic cytokine receptor family. The functional TSLPR complex is composed of TSLPR and IL-7Ra. TSLPR is also known as cytokine receptor-like molecule 2 or type I cytokine receptor σ1. TSLP initiates intracellular type 2 signaling by binding to its high affinity heterodimeric receptor complex, which consists of its specific receptor TSLPR, which has 24% homology to the co-receptor y chain in IL-2, IL-4, IL-9 and IL-15 and does not contain the δ-common chain IL-2 family, and the IL-7Rα subunit (CD127) in cells co-expressing TSLPR and IL-7Rα. TSLP initially binds to TSLPR and subsequently recruits the IL-7Rα chain.

### Pharmaceutical compositions

The present invention also provides a composition. Preferably, the composition is a pharmaceutical composition, which contains the aforementioned antibody or active fragment thereof or a fusion protein thereof, and a pharmaceutically acceptable carrier. Typically, these substances can be formulated in a non-toxic, inert and pharmaceutically acceptable aqueous carrier medium, where the pH is typically about 5-8, preferably about 6-8, although the pH may vary depending on the nature of the substance being formulated and the condition to be treated. The formulated pharmaceutical composition may be administered by conventional routes, which include (but are not limited to): intraperitoneal, intravenous, or topical administration.

The pharmaceutical compositions of the present invention can be used to bind directly to TSLP protein molecules and can therefore be used to treat diseases or conditions associated with TSLP (including immune system diseases or oncological diseases). In addition, other therapeutic agents can be used simultaneously.

The pharmaceutical compositions of the present invention contain a safe and effective amount (e.g., 0.001-99wt%, preferably 0.01-90wt%, more preferably 0.1-80wt%) of the nanobodies (or conjugates thereof) of the present invention as described above and a pharmaceutically acceptable carrier or excipient. Such carriers include (but are not limited to): saline, buffer, glucose, water, glycerol, ethanol, and combinations thereof. The pharmaceutical formulation should be matched to the mode of administration. The pharmaceutical compositions of the present invention may be made in the form of injections, for example prepared by conventional methods using saline or aqueous solutions containing glucose and other excipients. Pharmaceutical compositions such as injections and solutions are preferably manufactured under aseptic conditions. The active ingredient is administered in a therapeutically effective amount, e.g., about 10 µg/kg body weight - about 50 mg/kg body weight per day. In addition, the peptides of the invention can be used in combination with other therapeutic agents.

When using the pharmaceutical composition, a safe and effective amount of the Immunoconjugate is administered to the mammal, wherein the safe and effective amount is typically at least about 10 µg/kg body weight and in most cases does not exceed about 50 mg/kg body weight, preferably the dose is about 10 µg/kg body weight - about 10 mg/kg body weight. Of course, the exact dose should also take into account the route of administration, the health status of the patient, etc., which are all within the skill of the skilled practitioner.

### Anti-TSLP nanobodies

In the present invention, the amino acid sequence of the VHH chain of the anti-TSLP nanobody is selected from one or more of SEQ ID NO: 8, SEQ ID NO: 12, SEQ ID NO: 21, SEQ ID NO: 25, SEQ ID NO: 34 or SEQ ID NO: 38.

In a preferred embodiment of the present invention, the anti-TSLP nanobody comprises a monomer, a bivalent (bivalent antibody), a tetravalent (tetravalent antibody), and/or a multivalent (multivalent antibody).

Typically, the anti-TSLP nanobody comprises two VHH chains having an amino acid sequence as shown in SEQ ID NO: 8, SEQ ID NO: 12, SEQ ID NO: 21, SEQ ID NO: 25, SEQ ID NO: 34 or SEQ ID NO: 38.

In another preferred embodiment, the VHH chains are linked to each other by a linker peptide.

In a further preferred example, the linking peptide is selected from the sequence: (GₐS_{b})ₓ, wherein a, b, x = 0 or 1 or 2 or 3 or 4 or 5 or 6 or 7 or 8 or 9 or 10 (preferably, a = 4 and b = 1 and x = 4).

In another preferred example, the sequence of the linker peptide is
GGGGSGGGGSGGGGSGGGGS.

### Labelled nanobodies

In a preferred embodiment of the present invention, the nanobodies carry detectable markers. More preferably, the markers are selected from the group consisting of: isotopes, colloidal gold markers, coloured markers or fluorescent markers.

Colloidal gold markers can be carried out by methods known to those skilled in the art. In a preferred embodiment of the present invention, the nanobodies of TSLP are labelled with colloidal gold to obtain colloidal gold-labelled nanobodies.

### Detection methods

The present invention also relates to a method for detecting the TSLP protein. The steps for the methods are substantially as follows: obtaining a cell and/or tissue sample; lysing the sample in a medium; and detecting the level of TSLP protein in the lysed sample.

In the detection method of the present invention, the samples used are not particularly limited, representative examples being cell-containing samples present in cell preservation fluid.

### Kit

The invention also provides a kit containing an antibody (or fragment thereof) or detection plate of the invention and, in a preferred example of the invention, the kit further comprises a container, instructions for use, buffer and the like.

The invention also provides a detection kit for detecting TSLP levels, the kit comprising an antibody recognizing the TSLP protein, a lysis medium for lysing the sample, general reagent and buffer required for the assay, such as various buffer, detection marker, detection substrate, etc. The detection kit may be an in vitro diagnostic device.

### Applications

As described above, the nanobodies of the present invention have a wide range of biological applications and clinical applications in a variety of fields such as diagnosis and treatment of diseases or conditions associated with TSLP, basic medical research, biological research, etc. A preferred application is for clinical diagnosis and targeted therapy against TSLP.

The main advantages of the present invention include:
(a) The nanobodies of the present invention are effective in blocking the interaction of TSLP with TSLPR.
(b) The nanobodies of the present invention have stronger blocking activity compared to the control antibody Tezepelumab.
(c) The nanobodies of the present invention can be expressed in pichia pastoris with higher shake flask expression yields and fermenter yields up to 17-23 g/L.
(d) The nanobody of the present invention inhibits the pSTAT5 signaling pathway in BaF3/TSLPR-IL7R cells significantly better than the control antibody Tezepelumab.

The present invention is further described below in connection with specific embodiments. It should be understood that these embodiments are intended to illustrate the invention only and are not intended to limit the scope of the invention. Experimental methods for which specific conditions are not indicated in the following embodiments generally follow conventional conditions, such as those described in Sambrook et al, Molecular Cloning: A Laboratory Manual (New York: Cold Spring Harbor Laboratory Press, 1989), or as recommended by the manufacturer. Unless otherwise stated, percentages and parts are percentages by weight and parts by weight.

Materials and reagents used in embodiments of the present invention are commercially available products unless otherwise stated.

### Example 1 TSLP-specific nanobody screening

The amino acid sequence of human TSLP mutated at the furin site was optimized according to the human codon, the base sequence was cloned into the pFUSE vector, and the human TSLP protein was purified after transfection of HEK293F cells. The high purity protein was mixed with immunoadjuvant and four Xinjiang Bactrian camels were immunized once a week. After seven immunizations, peripheral blood was taken from the camels, RNA was isolated and the VHH gene fragment was amplified, which was then cloned into the pMECS vector and electrotransformed into TG1 competent cells to establish a high quality phage-displayed nano-antibody library. All four libraries are tested to have a library volume of more than 1x10⁹ CFU, and the fragment insertion rate is above 80%.

Screening of TSLP-specific nano-antibodies using phage display technology. After three rounds of "adsorption-wash-enrichment", specific nanobody phage enrichment occurs in all libraries. From these, 1200 clones were randomly selected for ELISA and sequencing analysis, the final 312 strains of differentially sequenced antibodies were obtained.

### Example 2 Flow cytometry screening of blocking TSLP nanobodies

Nanobody clones of the above sequences were inoculated in TB medium for the culture and induced overnight with IPTG, and supernatants were collected from lysed cells for blocking activity assays. The cultured CHOZEN/TSLPR stable cells were divided into 96-well plates with 3E5 cells per well, centrifuged at 3000 rpm for 3 min to remove the supernatant, incubated for 20 min with the lysate of each antibody and TSLP-Biotin protein, centrifuged and the supernatant discarded, added diluted SA-PE antibody and incubated for 20 min at 4°C. Centrifuge again and discard supernatant, add 200uL PBS per well to resuspend cells, flow cytometry was performed to detect PE signal of sample. The results show that there are 64 candidate antibodies with the function of blocking the interaction between TSLP and TSLPR. In combination with sequence analysis, 31 different families of antibodies are selected for expression purification. The purification method is described in Example 4 of patent CN110144011A.

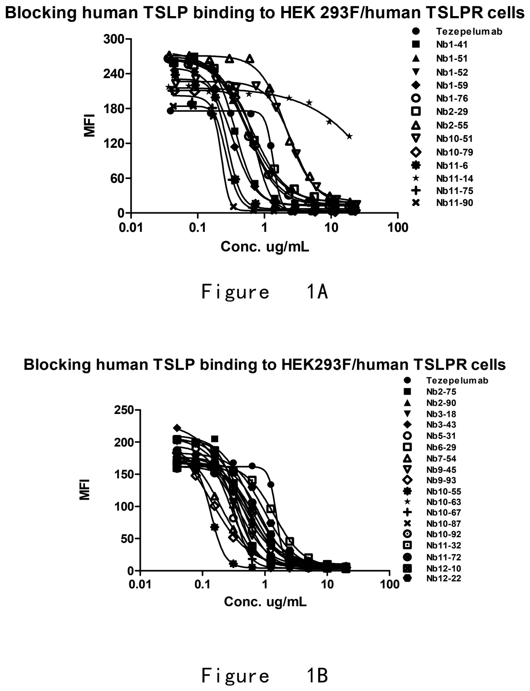

The above 31 purified antibodies were again subjected to flow cytometry blocking activity assay. The cultured HEK293F/TSLPR transient cells were divided into 96-well plates with 3E5 cells per well, centrifuged at 3000 rpm for 3 min to remove the supernatant, and incubated for 20 min with gradient-diluted antibodies (2-fold gradient dilution from 20ug/mL) and TSLP-Biotin protein. Tezepelumab was used as the control antibody in this experiment and then centrifuged and the supernatant discarded, diluted SA-PE antibody was added and incubated for 20 min at 4°C. Centrifuged again and discard supernatant, add 200uL PBS per well to resuspend cells, flow cytometry was performed to detect PE signal of sample. The results are shown in Figure 1A and 1B, where 14 antibodies show significantly better blocking activity than the control antibody Tezepelumab. The 14 antibodies are numbered as Nb1-41, Nb1-51, Nb1-59, Nb3-18, Nb3-43, Nb5-31, Nb6-29, Nb7-54, Nb10-55, Nb10-63, Nb10-87, Nb11-6, Nb11-72, Nb11-75.

### Example 3 Antibody affinity assay

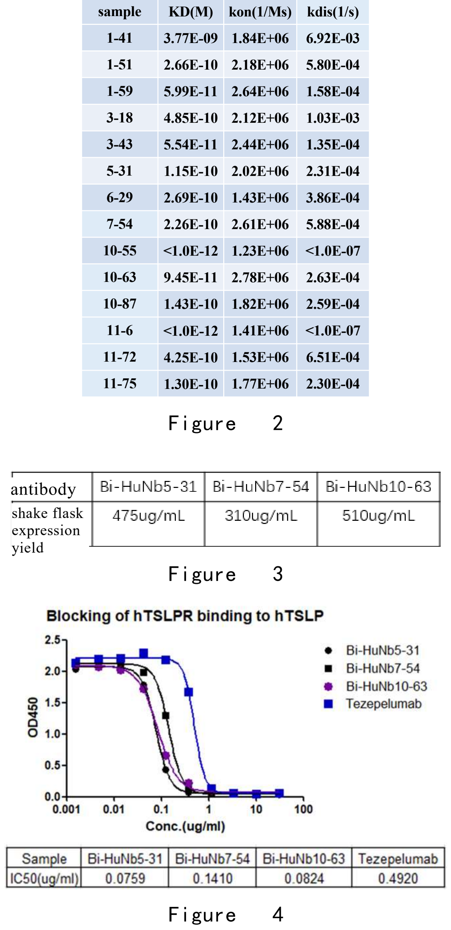

The binding kinetics of 14 blocking TSLP nanobodies were measured by Bio-layer interferometry (BLI). For kinetic measurements, candidate antibodies were diluted to 5ug/mL in PBST buffer, TSLP-Fc antigen diluted in PBST buffer in 2-fold gradients in six concentration gradients (2-fold gradient dilution from 20nM), and set the instrument operating conditions: Temperature 30°C, Shake speed 1000rpm. Antibodies were captured using a probe already encapsulated with Protein A, capture time 60s; Binding of gradient diluted antigen, binding time 240s; dissociation time 300s; 10mM glycine (pH 1.7) regenerated 2 times for 5s each. Fortebio Analysis version 9.0 was used to perform the analysis, Global mode was fitted and the binding rate (Kon), dissociation rate (Kdis) and dissociation constant KD were calculated. The results are shown in Figure 2.

### Example 4 Expression of humanized bivalent antibodies by Pichia pastoris

Humanization of the candidate antibody, keeping the variable region intact and humanized design for four framework region sequences, see the method of Example 4 in patent CN2018101517526 for the modification method. The amino acid sequences of the humanized antibodies are shown in Table 1.

| Table 1 Antibody regions | Nb10-63 Serial number (SEQ ID NO:) | | Nb7-54 Serial number (SEQ ID NO:) | | Nb5-31 Serial number (SEQ ID NO:) | |
|---|---|---|---|---|---|---|
| | Before humanization | After humanization | Before humanization | After humanization | Before humanization | After humanization |
| FR1 | 4 | 10 | 17 | 23 | 30 | 36 |
| CDR1 | 1 | 1 | 14 | 14 | 27 | 27 |
| FR2 | 5 | 5 | 18 | 18 | 31 | 31 |
| CDR2 | 2 | 2 | 15 | 15 | 28 | 28 |
| FR3 | 6 | 6 | 19 | 19 | 32 | 32 |
| CDR3 | 3 | 3 | 16 | 16 | 29 | 29 |
| FR4 | 7 | 11 | 20 | 24 | 33 | 37 |
| Complete amino acid sequence | 8 | 12 | 21 | 25 | 34 | 38 |
| Complete nucleotide sequence | 9 | 13 | 22 | 26 | 35 | 39 |

The above humanized antibodies were constructed in bivalent form and ligated using linker (G₄S) 4. The sequences after ligation are shown in SEQ ID NO.:40, SEQ ID NO.:41 and SEQ ID NO.:42 and were subsequently expressed using pichia pastoris. Briefly, expression was performed as follows: (1) the above TSLP nanobody bivalent sequence was constructed into the pPICZaA vector; (2) electrotransformed into X-33 competent cells after linearization with Sac I restriction endonuclease; (3) the electrotransformed samples were individually coated on YPD plate medium containing different concentrations of bleomycin resistance and incubated in an incubator at 30°C for 3-4 days; (4) After the monoclonal clone has grown on the plate medium, pick the monoclonal clone on the plates with different concentrations and place them in BMMY medium, when the OD value of BMMY medium reaches about 20, collect the bacterium and replace it into BMMY medium, incubated at 28°C, 250rpm; (5) after that, take samples every 24h and make up the final volume of 1% methanol and take samples; samples were centrifuged at 12000rpm for 5min, remove supernatant and stored at -20°C; induced for 5 consecutive days and end the incubation; (6) The supernatant samples taken were subjected to SDS-PAGE detection. The shake flask expression of the bivalent single domain antibody is shown in Figure 3.

### Example 5 ELISA to detect humanized bivalent antibody blocking activity

The above purified bivalent humanized antibodies were subjected to ELISA to detect blocking activity. Human TSLP antigen protein was dispensed into 96-well plates and incubated overnight at 4°C; after washing 5 times with PBST, 300uL of 1% BSA blocking solution was added and incubated at 37°C for 2 hours; after washing 5 times with PBST, 50uL of gradient-diluted antibody samples were added, followed by 50uL of 0.02ug/mL biotinylated TSLPR protein per well and incubated at 37°C for 1 hour; After washing 5 times with PBST, 100uL of SA-HRP (1: 100000 dilution) was added and incubated for 1 hour at 37°C. After washing 5 times with PBST, 100uL of TMB color-substrate solution was added and colour development at 37°C for 10min. The reaction was terminated by adding 2M H₂SO₄ 50uL/well, and the absorption value was measured at 450nm on an microplate reader. The results are shown in Figure 4: the blocking activity of the three humanized bivalent antibodies is significantly better than that of the control antibody Tezepelumab.

### Example 6 Inhibition of the pSTAT5 signaling pathway in BaF3/TSLPR-IL7R cells by humanized bivalent antibodies

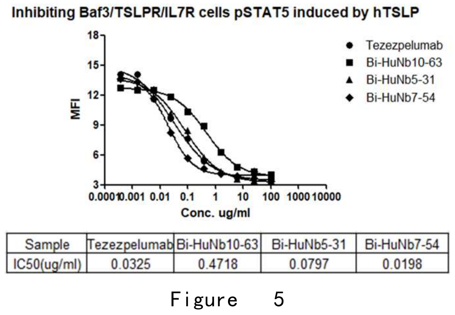

Well-grown BaF3/TSLPR-IL7R cells were centrifuged at 1000rpm for 5min, resuspended in PBS and dispensed into 96-well plates at 1x10⁵ cells per well. 25uL of diluted human TSLP factor and gradient dilution of bivalent humanized antibody or Tezepelumab were mixed, incubated at 37 degrees for 30 min and then the mixture was added to the cells at 37 degrees for 20 min at 5% CO₂. After washing the cells in PBS, formaldehyde was added to fix them and then pre-cooled methanol was added for 10 min of incubation. Finally, anti-phosphorylated STAT5-PE labelled antibody was added and incubated for 30 minutes. The cells were washed and the PE signal values were detected by flow cytometry. The results are shown in Figure 5, in which the two humanized antibodies inhibit the pSTAT5 signaling pathway in BaF3/TSLPR-IL7R cells significantly better than the control antibody Tezepelumab.

### Example 7 Evaluation of the yield of humanized bivalent antibodies in a 7L fermenter

The three antibody-expressing strains of glycerol were amplified at 1:100 and cultured for primary seeds, then transferred to fresh medium for the culture of secondary seeds, and then transferred to a 7L fermenter for fermentation culture after the secondary seeds were qualified. Automatic flow with ammonia to adjust the pH of the fermentation culture to 6.0. During the incubation process, samples are taken regularly to check the pH of the fermentation broth, wet weight of bacterial cell and OD value of bacteria solution and the stirring speed, tank pressure and oxygen ventilation capacity are adjusted according to the changes in dissolved oxygen. According to the change of wet weight and dissolved oxygen of fermenting bacterial cell, glycerol supplement medium and methanol supplement medium were added at different stages of fermentation culture. Methanol induced incubation for 160h to 200h and fermentation was stopped. The yields of the above three humanized bivalent antibodies are tested to be 17 g/L, 19 g/L and 23 g/L, respectively. The antibody yields are much higher than the fermentation expression yields of all types of antibodies that have been reported, reaching an industry leading level.

### Sequence information:

SEQ ID NO.1 :
   GFTLDDSDMG
SEQ ID NO.2 :
   ISSLGGT
SEQ ID NO.3 :
   APGTDRYSDCPNEYSV
SEQ ID NO.4 :
   QVQLQESGGGSVQAGGSLRLSCTAS
SEQ ID NO.5 :
   WYRQAPGDECELVST
SEQ ID NO.6:
   YYADSVKGRFTISHDNAKNTVYLQMNSLKPHDTAVYYC
SEQ ID NO.7 :
   WGQGTQVTVSS
SEQ ID NO.8: 
QVQLQESGGGSVQAGGSLRLSCTASGFTLDDSDMGWYRQAPGDECELVSTI
SSLGGTYYADSVKGRFTISHDNAKNTVYLQMNSLKPHDTAVYYCAPGTDRYS
DCPNEYSVWGQGTQVTVSS
SEQ ID NO.9: 
CAGGTGCAGCTGCAGGAGTCTGGAGGAGGCTCGGTGCAGGCTGGAGGG
TCTCTGAGACTCTCCTGTACAGCCTCTGGATTCACTTTGGATGATTCTGACAT
GGGCTGGTACCGCCAGGCTCCAGGGGATGAGTGCGAGTTGGTCTCAACTAT
TAGTAGTTTGGGTGGCACATACTATGCAGACTCCGTGAAGGGCCGATTCACC
ATCTCCCATGACAACGCCAAGAACACGGTGTATCTGCAAATGAACAGCCTG
AAACCTCACGACACGGCCGTGTATTACTGTGCGCCGGGACAGACCGTTAT
AGCGACTGCCCTAATGAGTATAGCGTCTGGGGCCAGGGGACCCAGGTCACC
GTCTCCTCA
SEQ ID NO.10:
   EVQLLESGGGLVQPGGSLRLSCTAS
SEQ ID NO.11:
   WGQGTLVTVSS
SEQ ID NO.12: 
EVQLLESGGGLVQPGGSLRLSCTASGFTLDDSDMGWYRQAPGDECELVSTI
SSLGGTYYADSVKGRFTISHDNAKNTVYLQMNSLKPHDTAVYYCAPGTDRYS
DCPNEYSVWGQGTLVTVSS
SEQ ID NO.13: 
GAGGTTCAATTGTTGGAATCTGGTGGTGGTTTGGTTCAACCAGGTGGTTC
TTTGAGATTGTCTTGTACTGCTTCTGGTTTCACCTTGGACGATTCTGATATGG
GATGGTACAGACAAGCTCCAGGAGATGAGTGTGAGTTGGTTTCTACTATCTC
TTCCTTGGGTGGAACCTACTACGCTGATTCTGTCAAGGGTCGTTTCACTATTT
CTCACGATAATGCTAAGAACACCGTTTACTTGCAAATGAACTCTTTAAAGCC
ACATGATACTGCCGTTTACTACTGTGCTCCTGGTACTGATAGATACTCTGAC
TGTCCAAACGAATACTCCGTTTGGGGTCAGGGTACTTTGGTTACTGTCTCTTC
C
SEQ ID NO.14:
   GFTFDDSDMG
SEQ ID NO.15:
   ISSDGMT
SEQ ID NO.16:
   AATKYSSDYDVAEDWRRGVCGDMDY
SEQ ID NO.17:
   QVQLQESGGGSVQAGETLRLSCTAS
SEQ ID NO.18:
   WYRQAPGNECELVSI
SEQ ID NO.19:
   YYADSVKGRFTISQDNAKNTVYLQMNSLKPEDTAVYYC
SEQ ID NO.20:
   WGKGTQVTVSS
SEQ ID NO.21: 
QVQLQESGGGSVQAGETLRLSCTASGFTFDDSDMGWYRQAPGNECELVSII
SSDGMTYYADSVKGRFTISQDNAKNTVYLQMNSLKPEDTAVYYCAATKYSSD
YDVAEDWRRGVCGDMDYWGKGTQVTVSS
SEQ ID NO.22: 
CAGGTGCAGCTGCAGGAGTCTGGAGGAGGCTCGGTGCAGGCTGGAGAG
ACTCTGAGACTCTCCTGTACAGCCTCTGGATTCACTTTTGATGATTCTGACAT
GGGCTGGTACCGCCAGGCTCCAGGGAATGAGTGCGAGTTGGTCTCAATTATT
AGTAGTGATGGTATGACATACTATGCAGACTCCGTGAAGGGCCGATTCACC
 
ATCTCCCAAGACAACGCCAAGAACACGGTGTATCTGCAAATGAACAGCCTG
AAACCTGAGGACACAGCCGTGTATTACTGTGCGGCGACGAAGTACTCCAGC
GACTATGACGTAGCTGAGGATTGGAGACGCGGGGTCTGTGGAGACATGGAC
TACTGGGGCAAAGGAACCCAGGTCACCGTCTCCTCA
SEQ ID NO.23:
   EVQLLESGGGLVQPGETLRLSCTAS
SEQ ID NO.24:
   WGKGTLVTVSS
SEQ ID NO.25: 
EVQLLESGGGLVQPGETLRLSCTASGFTFDDSDMGWYRQAPGNECELVSIIS
SDGMTYYADSVKGRFTISQDNAKNTVYLQMNSLKPEDTAVYYCAATKYSSDY
DVAEDWRRGVCGDMDYWGKGTLVTVSS
SEQ ID NO.26: 
GAAGTCCAATTGCTTGAATCCGGTGGTGGATTAGTTCAACCAGGTGAGA
CCTTGAGACTGTCCTGTACCGCTTCCGGTTTCACTTTCGACGACTCCGACAT
GGGTTGGTACAGACAGGCTCCAGGTAATGAGTGTGAGTTGGTTTCTATTATT
TCCTCTGATGGTATGACTTACTACGCTGATTCTGTTAAGGGTAGATTCACTAT
CTCTCAAGACAATGCTAAGAACACTGTTTACTTGCAAATGAACTCTTTGAAG
CCTGAAGATACCGCCGTCTACTACTGTGCTGCCACCAAGTACTCCTCCGATT
ATGATGTCGCTGAAGATTGGAGAAGAGGAGTTTGTGGAGATATGGATTACT
GGGGTAAAGGTACTTTGGTTACCGTTTCTTCT
SEQ ID NO.27:
   GFTSGGSDMG
SEQ ID NO.28:
   ISSDGST
SEQ ID NO.29:
   AATDYGLGPPPSSTGQCYGMDY
SEQ ID NO.30:
   QVQLQESGGGSVQAGGSLRLSCTAS
SEQ ID NO.31:
   WYRQAPGNECDLVSY
SEQ ID NO.32:
   YYADSVKGRFTISQDNAKNTVYLQMNSLKPEDTAVYYC
SEQ ID NO.33:
   WGKGTQVTVSS
SEQ ID NO.34: QVQLQESGGGSVQAGGSLRLSCTASGFTSGGSDMGWYRQAPGNECDLVSY 
ISSDGSTYYADSVKGRFTISQDNAKNTVYLQMNSLKPEDTAVYYCAATDYGLG
PPPSSTGQCYGMDYWGKGTQVTVSS
SEQ ID NO.35: 
CAGGTGCAGCTGCAGGAGTCTGGGGGAGGCTCGGTGCAGGCTGGAGGG
TCTCTGAGACTCTCCTGTACAGCCTCTGGATTCACTTCTGGCGGTTCTGACAT
GGGCTGGTACCGCCAGGCTCCAGGGAATGAGTGCGACTTGGTCTCATATATT
AGTAGTGATGGTAGCACATACTATGCAGACTCCGTGAAGGGCCGATTCACC
ATCTCCCAAGACAACGCCAAGAACACGGTGTATCTGCAAATGAACAGCCTG
AAACCTGAGGACACGGCCGTGTATTACTGTGCGGCCACCGACTATGGGTTG
GGGCCGCCCCCTTCTTCGACGGGCCAATGTTACGGCATGGACTACTGGGGCA
AAGGAACCCAGGTCACCGTCTCCTCA
SEQ ID NO.36:
   EVQLLESGGGLVQPGGSLRLSCTAS
SEQ ID NO.37:
   WGKGTLVTVSS
SEQ ID NO.38: 
EVQLLESGGGLVQPGGSLRLSCTASGFTSGGSDMGWYRQAPGNECDLVSYI
SSDGSTYYADSVKGRFTISQDNAKNTVYLQMNSLKPEDTAVYYCAATDYGLGP
PPSSTGQCYGMDYWGKGTLVTVSS
SEQ ID NO.39: 
GAAGTTCAGTTGTTGGAATCAGGTGGTGGTTTGGTTCAACCAGGAGGTT
CTCTGAGATTGTCTTGTACTGCTTCCGGTTTCACTTCCGGAGGTTCTGACATG
GGATGGTACCGTCAAGCTCCTGGTAACGAGTGCGACTTGGTTTCTTACATCT
CTTCCGACGGTTCCACTTACTACGCTGATTCTGTTAAGGGTAGATTCACTATT
TCTCAAGACAACGCTAAGAATACTGTTTACTTGCAGATGAACTCTTTGAAGC
CAGAGGATACCGCTGTTTACTATTGTGCCGCCACTGATTACGGTTTGGGTCC
TCCACCATCTTCTACTGGACAATGTTACGGTATGGATTACTGGGGTAAAGGT
ACCCTGGTCACCGTTTCCTCT
SEQ ID NO.40: 
EVQLLESGGGLVQPGGSLRLSCTASGFTLDDSDMGWYRQAPGDECELVSTI
SSLGGTYYADSVKGRFTISHDNAKNTVYLQMNSLKPHDTAVYYCAPGTDRYS
DCPNEYSVWGQGTLVTVSSGGGGSGGGGSGGGGSGGGGSEVQLLESGGGLVQ
PGGSLRLSCTASGFTLDDSDMGWYRQAPGDECELVSTISSLGGTYYADSVKGR
FTISHDNAKNTVYLQMNSLKPHDTAVYYCAPGTDRYSDCPNEYSVWGQGTLV
TVSS
SEQ ID NO.41: EVQLLESGGGLVQPGETLRLSCTASGFTFDDSDMGWYRQAPGNECELVSIIS 
SDGMTYYADSVKGRFTISQDNAKNTVYLQMNSLKPEDTAVYYCAATKYSSDY
DVAEDWRRGVCGDMDYWGKGTLVTVSSGGGGSGGGGSGGGGSGGGGSEVQ
LLESGGGLVQPGETLRLSCTASGFTFDDSDMGWYRQAPGNECELVSIISSDGMT
YYADSVKGRFTISQDNAKNTVYLQMNSLKPEDTAVYYCAATKYSSDYDVAED
WRRGVCGDMDYWGKGTLVTVSS
SEQ ID NO.42:

EVQLLESGGGLVQPGGSLRLSCTASGFTSGGSDMGWYRQAPGNECDLVSYI
SSDGSTYYADSVKGRFTISQDNAKNTVYLQMNSLKPEDTAVYYCAATDYGLGP
PPSSTGQCYGMDYWGKGTLVTVSSGGGGSGGGGSGGGGSGGGGSEVQLLESG
GGLVQPGGSLRLSCTASGFTSGGSDMGWYRQAPGNECDLVSYISSDGSTYYAD
SVKGRFTISQDNAKNTVYLQMNSLKPEDTAVYYCAATDYGLGPPPSSTGQCYG
MDYWGKGTLVTVSS

All publications mentioned herein are incorporated by reference as if each individual document was cited as a reference, as in the present application. It should also be understood that, after reading the above teachings of the present invention, those skilled in the art can make various changes or modifications, equivalents of which falls in the scope of claims as defined in the appended claims.
The following numbered clauses, describing aspects of our proposals, are part of the description
1. An anti-TSLP nanobody, wherein the complementary determining region CDR region of the VHH chain in the nanobody is one or more selected from the group consisting of:
   (1) CDR1 as shown in SEQ ID NO:1, CDR2 as shown in SEQ ID NO:2, and CDR3 as shown in SEQ ID NO: 3;
   (2) the CDR1 shown in SEQ ID NO:14, the CDR2 as shown in SEQ ID NO:15, and the CDR3 as shown in SEQ ID NO: 16; and
   (3) the CDR1 as shown in SEQ ID NO:27, the CDR2 as shown in SEQ ID NO:28, and the CDR3 as shown in SEQ ID NO: 29.
2. The anti-TSLP nanobody of clause 1, wherein the VHH chain of the anti-TSLP nanobody further comprises a framework region FR, the framework region FR is one or more selected from the group consisting of:
   (1) FR1 as shown in SEQ ID NO: 4, FR2 as shown in SEQ ID NO: 5, FR3 as shown in SEQ ID NO: 6, and FR4 as shown in SEQ ID NO: 7;
   (2) FR1 as shown in SEQ ID NO: 10, FR2 as shown in SEQ ID NO: 5, FR3 as shown in SEQ ID NO: 6, and FR4 as shown in SEQ ID NO: 11;
   (3) FR1 as shown in SEQ ID NO: 17, FR2 as shown in SEQ ID NO: 18, FR3 as shown in SEQ ID NO: 19, and FR4 as shown in SEQ ID NO: 20;
   (4) FR1 as shown in SEQ ID NO: 23, FR2 as shown in SEQ ID NO: 18, FR3 as shown in SEQ ID NO: 19, and FR4 as shown in SEQ ID NO: 24;
   (5) FR1 as shown in SEQ ID NO: 30, FR2 as shown in SEQ ID NO: 31, FR3 as shown in SEQ ID NO: 32, and FR4 as shown in SEQ ID NO: 33; and
   (6) FR1 as shown in SEQ ID NO: 36, FR2 as shown in SEQ ID NO: 31, FR3 as shown in SEQ ID NO: 32, and FR4 as shown in SEQ ID NO: 37.
3. The anti-TSLP nanobody of clause 1, wherein the amino acid sequence of the VHH chain of the anti-TSLP nanobody is selected from the group consisting of SEQ ID NO: 8, SEQ ID NO: 12, SEQ ID NO: 21, SEQ ID NO: 25, SEQ ID NO: 34, SEQ ID NO: 38, and a combination thereof.
4. An anti-TSLP antibody which is directed against a TSLP epitope and has an anti-TSLP nanobody of clause 1.
5. An anti-TSLP antibody comprising one or more anti-TSLP nanobodies of clause 1.
6. The antibody of clause 4 or 5, wherein the antibody comprises a monomer, a bivalent antibody, and/or a multivalent antibody.
7. A polynucleotide encoding a protein selected from the group consisting of: an anti-TSLP nanobody of clause 1 or an anti-TSLP antibody of clause 4 or an anti-TSLP antibody of clause 5.
8. An expression vector, comprising a polynucleotide of clause 7.
9. A host cell containing the expression vector of clause 8, or having the polynucleotide of clause 7 integrated in its genome.
10. A method for producing an anti-TSLP nanobody, comprising the steps of:
   (a) culturing the host cell of clause 9 under a condition suitable for producing a nanobody, thereby obtaining a culture containing the anti-TSLP nanobody;
   (b) isolating or recovering the anti-TSLP nanobody from the culture; and
   (c) optionally, purifying and/or modifying the anti-TSLP nanobody obtained in step (b).
11. An immunoconjugate, comprising:
   (a) the anti-TSLP nanobody of clause 1, or the anti-TSLP antibody of clause 4 or the anti-TSLP antibody of clause 5 ; and
   (b) a coupling portion selected from the group consisting of: a detectable marker, a drug, a toxin, a cytokine, a radionuclide, an enzyme, a gold nanoparticle/nanorod, a magnetic nanoparticle, a viral capsid protein or VLP, and a combination thereof.
12. A multispecific antibody, wherein the multispecific antibody comprising: an anti-TSLP nanobody of clause 1, or an anti-TSLP antibody of clause 4 or an anti-TSLP antibody of clause 5.
13. A recombinant protein, wherein the recombinant protein having:
   (i) an anti-TSLP nanobody of clause 1, an anti-TSLP antibody of clause 4 or an anti-TSLP antibody of clause 5; and
   (ii) optionally a tag sequence to assist in the expression and/or purification.
14. A pharmaceutical composition, comprising:
   (i) an anti-TSLP nanobody of clause 1, or an anti-TSLP antibody of clause 4 or an anti-TSLP antibody of clause 5, or an immunoconjugate of clause 11 or a multispecific antibody of clause 12 or a recombinant protein of clause 13; and
   (ii) a pharmaceutically acceptable vector.
15. Use of an anti-TSLP nanobody of clause 1, an anti-TSLP antibody of clause 4 or an anti-TSLP antibody of clause 5, or an immunoconjugate of clause 11 or a multispecific antibody of clause 12 or a recombinant protein of clause 13 or a pharmaceutical composition of clause 14 (a) for the preparation of a drug for the prevention and/or treatment of a disease associated with TSLP; and/or (b) for the preparation of a reagent, a detection plate or a kit for the detection of TSLP.
16. A method for detecting TSLP protein in a sample, comprising the steps of:
   (1) contacting a sample with an anti-TSLP nanobody of clause 1, an anti-TSLP antibody of clause 4 or an anti-TSLP antibody of clause 5, or an immunoconjugate of clause 11 or a multispecific antibody of clause 12 or a recombinant protein of clause 13; and
   (2) detecting the formation of an antigen-antibody complex, wherein the formation of a complex indicates the presence of TSLP protein in the sample.
17. A TSLP protein detection reagent, comprising:
   (i) an anti-TSLP nanobody of clause 1, an anti-TSLP antibody of clause 4 or an anti-TSLP antibody of clause 5, or an immunoconjugate of clause 11 or a multispecific antibody of clause 12 or a recombinant protein of clause 13; and
   (ii) a vector that is acceptable for the detection.
18. A kit for detecting a TSLP protein, containing an immunoconjugate of clause 7 or a detection reagent of clause 17, and instructions.
19. Use of an immunoconjugate of clause 17 for the preparation of a contrast agent for the in vivo detection of TSLP protein.

## Claims

1. An anti-thymic stromal lymphopoietin (TSLP) single domain antibody, wherein the complementary determining regions (CDRs) of the VHH chain in the single domain antibody comprise:
CDR1 as shown in SEQ ID NO:1, CDR2 as shown in SEQ ID NO:2, and CDR3 as shown in SEQ ID NO: 3.

2. The anti-TSLP single domain antibody of claim 1, wherein the VHH chain of the anti-TSLP single domain antibody further comprises a framework region FR, the framework region FR is one or more selected from the group consisting of:
(1) FR1 as shown in SEQ ID NO: 4, FR2 as shown in SEQ ID NO: 5, FR3 as shown in SEQ ID NO: 6, and FR4 as shown in SEQ ID NO: 7; and
(2) FR1 as shown in SEQ ID NO: 10, FR2 as shown in SEQ ID NO: 5, FR3 as shown in SEQ ID NO: 6, and FR4 as shown in SEQ ID NO: 11.

3. The anti-TSLP single domain antibody of claim 1 or claim 2, wherein the amino acid sequence of the VHH chain of the anti-TSLP single domain antibody is selected from the group consisting of SEQ ID NO: 8, SEQ ID NO: 12, and a combination thereof.

4. An anti-TSLP antibody which is directed against a TSLP epitope and has one or more of the anti-TSLP single domain antibodies of any one of claims 1 to 3, wherein the antibody optionally comprises a monomer, a bivalent antibody, and/or a multivalent antibody.

5. A polynucleotide encoding a protein selected from the group consisting of: the anti-TSLP single domain antibody of claim 1 or the anti-TSLP antibody of claim 4.

6. An expression vector, comprising the polynucleotide of claim 5.

7. A host cell containing the expression vector of claim 6, or having the polynucleotide of claim 5 integrated in its genome.

8. A method for producing an anti-TSLP single domain antibody, comprising the steps of:
(a) culturing the host cell of claim 7 under a condition suitable for producing a single domain antibody, thereby obtaining a culture containing the anti-TSLP single domain antibody;
(b) isolating or recovering the anti-TSLP single domain antibody from the culture; and
(c) optionally, purifying and/or modifying the anti-TSLP single domain antibody obtained in step (b).

9. An immunoconjugate, comprising:
(a) the anti-TSLP single domain antibody of claim 1, or the anti-TSLP antibody of claim 4; and
(b) a coupling portion selected from the group consisting of: a detectable marker, a drug, a toxin, a cytokine, a radionuclide, an enzyme, a gold nanoparticle/nanorod, a magnetic nanoparticle, a viral capsid protein or VLP, and a combination thereof.

10. A multispecific antibody, wherein the multispecific antibody comprises the anti-TSLP single domain antibody of claim 1, or the anti-TSLP antibody of claim 4.

11. A recombinant protein, the recombinant protein having:
(i) the anti-TSLP single domain antibody of claim 1, or the anti-TSLP antibody of claim 4; and
(ii) optionally a tag sequence to assist in the expression and/or purification.

12. A pharmaceutical composition, comprising:
(i) the anti-TSLP single domain antibody of claim 1, or the anti-TSLP antibody of claim 4, or the immunoconjugate of claim 9, or the multispecific antibody of claim 10, or the recombinant protein of claim 11; and
(ii) a pharmaceutically acceptable carrier.

13. The anti-TSLP single domain antibody of claim 1, the anti-TSLP antibody of claim 4, the immunoconjugate of claim 9, the multispecific antibody of claim 10, the recombinant protein of claim 11, or the pharmaceutical composition of claim 12 for use (a) in a method of prevention and/or treatment of a disease associated with TSLP; and/or (b) in the preparation of a reagent, a detection plate or a kit for the detection of TSLP.

14. A method for detecting TSLP protein in a sample, comprising the steps of:
(1) contacting a sample with the anti-TSLP single domain antibody of claim 1, the anti-TSLP antibody of claim 4, the immunoconjugate of claim 9, the multispecific antibody of claim 10, or the recombinant protein of claim 11; and
(2) detecting the formation of an antigen-antibody complex, wherein the formation of a complex indicates the presence of TSLP protein in the sample.

15. A TSLP protein detection reagent, comprising:
(i) the anti-TSLP single domain antibody of claim 1, the anti-TSLP antibody of claim 4, the immunoconjugate of claim 9, the multispecific antibody of claim 10, or the recombinant protein of claim 11; and
(ii) a carrier that is acceptable for the detection.

16. A kit for detecting a TSLP protein, containing the immunoconjugate of claim 9 or the detection reagent of claim 15, and instructions.

17. The immunoconjugate of claim 9 for use in the preparation of a contrast agent for the *in vivo* detection of TSLP protein.
